# EUROPÄISCHE PATENTSCHRIFT

(11) **EP 0 679 678 B1**
(45) Veröffentlichungstag und Bekanntmachung des Hinweises auf die Patenterteilung: **05.01.2000**
(21) Anmeldenummer: 95104872.7
(22) Anmeldetag: 01.04.1995
(51) Int. Cl.: C08J 3/12, B01J 20/26, B01J 20/32, A61L 15/00

(54) **Staubarme pulverförmige hydrophile Polymere**
Dust-free powdery hydrophilic polymers
Polymères hydrophiles pulverulents sans poussière

(30) Priorität: 22.04.1994 DE 4414117
(43) Veröffentlichungstag der Anmeldung: 02.11.1995
(73) Patentinhaber: BASF AKTIENGESELLSCHAFT, 67056 Ludwigshafen (DE)
(72) Erfinder: Klotzsche, Helmut, D-63755 Alzenau (DE); Remmel, Gustav, D-63571 Gelnhausen (DE); Riegel, Ulrich, D-60386 Frankfurt am Main (DE); Stüven, Uwe, D-65812 Bad Soden (DE)

(56) Entgegenhaltungen:
- EP-A- 0 096 790
- EP-A- 0 224 923
- EP-A- 0 509 708
- WO-A-94/22940
- FR-A- 2 646 430
- US-A- 4 080 358

## Beschreibung

Die vorliegende Erfindung betrifft pulverförmige wasserquellbare hydrophile Polymerisate, wie sie z.B. zur Absorption von Wasser und wäßrigen Lösungen, insbesondere Körperflüssigkeiten wie Blut und Urin, eingesetzt werden, denen zur Verringerung des Staubens ein oder mehrere Hilfsstoffe zugesetzt wurden, -deren Dampfdrucke bei 20°C höchstens 0,1 mbar betragen und deren Schmelzpunkte maximal bei 100°C liegen.

Derartige wasserabsorbierende Polymere, vielfach auch Superabsorber genannt, sind an sich durch zahlreiche Veröffentlichungen bekannt. Es kommen modifizierte natürliche Polymere und teilweise oder vollständig synthetische Polymere zum Einsatz. Die vollsynthetischen Polymeren werden in der Regel durch radikalische Polymerisation verschiedener hydrophiler Monomerer in wäßriger Lösung nach unterschiedlichen Methoden hergestellt. Im allgemeinen werden dabei Vernetzer einpolymerisiert, wodurch das erhaltene Polymer nicht mehr wasserlöslich, sondern nur noch wasserquellbar ist. Als Superabsorber können z.B. Polymere auf Basis von (Meth-)Acrylsäure verwendet werden, die teilweise in neutralisierter Form als Alkalisalze vorliegen.

Das Superabsorber-Polymer wird in der Regel mechanisch zerkleinert, getrocknet und gemahlen. Dabei fällt das pulverförmige wasserquellbare Polymer je nach Herstellungsverfahren in einem mehr oder weniger breiten Kornspektrum an. Ein typisches Kornspektrum für ein nach der Trocknung vermahlenes wasserquellbares vollsynthetisches Polymer liegt im Bereich von 10 bis 900 µm, wovon die Kornfraktion von 100 bis 850 µm für praktische Zwecke als Absorptionsmaterial eingesetzt wird. Trotz Absiebens über ein Siebdeck mit einer Maschenweite von 150 µm und Abtrennen des Feinstaubes einer Korngröße bis etwa 150 µm können in der Ware, die zum Einsatz als Absorptionsmaterial vorgesehen ist, unter technischen Bedingungen immer nqch Restanteile an Feinstaub einer Korngröße kleiner 100 µm bis zu 1 % und einer Korngröße kleiner 10 µm bis zu 0,5 % verbleiben. Feinstaub einer Korngröße kleiner 10 µm ist aus inhalationstoxikologischen Gründen unerwünscht. Feinstaubanteile kleiner 100 µm verursachen das visuell sichtbare Stauben mit all seinen Folgeerscheinungen und führen zu Handlingsproblemen im Produktions- und im Verarbeitungsbetrieb und sind daher ebenfalls unerwünscht.

Die Behandlung von partikelförmigen Stoffen zur Verringerung oder Unterbindung des Staubens ist an sich aus zahlreichen Veröffentlichungen bekannt. Z.B. ist in der tschechoslowakischen Patentschrift CS B-237 182 die Zugabe von 2 bis 5 % verschiedener Hilfsstoffe zu Tanninen zur Staubverhinderung beschrieben, in der japanischen Patentanmeldung JP A-03/247644 die Zugabe von 0,2 bis 2,0 Teilen Siliconöl zu Polyvinylchlorid, in der japanischen Patentanmeldung JP A-01/271485 das Aufsprühen von polyether-modifizierten Siliconölen auf feste Brennstoffe, wie z.B. Kohle, in der finnischen Patentschrift FI-B-85387 die Behandlung von Mineralwollprodukten mit polymeren Antistaubmitteln, wie z.B. Polyvinylacetat, in der europäischen Patentanmeldung EP-A-570881 die Herstellung staubarmer Granulate durch Aufbringen einer Lösung oder Suspension einer hydratbildenden Substanz, wie z.B. Natriumsulfat, oder in den deutschen Offenlegungsschriften DE-A-28 45 975 und DE-A-30 48 940 die Behandlung von Perliten mit Siliconölen zur Hydrophobierung für die Verwendung als Ölabsorptionsmittel und zur Verhinderung des Staubens. In allen diesen Fällen handelt es sich bei den Substanzen, die zur Verhinderung oder Verringerung des Staubens mit verschiedenen Hilfsstoffen behandelt werden, aber nicht um wasserquellbare hydrophile Polymere.

In der WO-A-90/09236 werden Bindemittel für Flüssigkeiten beschrieben, die Superabsorber und - zur Vermeidung des Staubens - auch Polyethylenglykole enthalten; es handelt sich dabei aber um Abmischungen von Superabsorbern mit anorganischen oder organischen Verbindungen mit großer Oberfläche und/oder kapillarem und/oder faserigem Aufbau, die mit einer relativ großen Menge Polyethylenglykol versetzt werden, beispielsweise 20 % Polyethylenglykol, bezogen auf das Gewicht des Superabsorber-Polymers. Teil- und vollsynthetische Absorptionsmittel, denen zur Verbesserung der Blutdispergierbarkeit und Blut-Netzbarkeit Polyether (Polyethylenglykole, Polypropylenglykole, Polyethylenoxid/Polypropylenoxid-Blockpolymerisate) oder Kohlenwasserstoffe, Fettalkohole, Fettsäuren oder Fettsäureester zugesetzt sind, sind in der DE-A-28 44 956 bzw. der EP-B-9977 beschrieben, auch hier werden aber, insbesondere im Falle vollsynthetischer Absorptionsmittel auf Basis Polyacrylsäure, relativ große Mengen der Hilfsstoffe zugesetzt; über eine Verringerung des Staubens wird hier aber nichts berichtet. Die Agglomerierung von pulverförmigen wasserquellbaren Polymeren zur Verhinderung des Blockens und zur Verbesserung der Flüssigkeitsaufnahmegeschwindigkeit wird in der DE-A-37 41 157 und 37 41 158 beschrieben, jedoch handelt es sich hier um die Agglomerierung von ausschließlich feinkörnigem Polymer, z.B. solchem mit mehr als 90 Gew.% Kornanteil unter 90 µm. Die Agglomerierung von Polymerteilchen mit einem mittleren Teilchendurchmesser von 0,1 bis 15 µm mit bestimmten Polyalkylenglykol-Derivaten wird in der WO-A-92/13912 offenbart. Ein Verfahren zur Umwandlung von ebenfalls ausschließlich Feinstpulvern wasserquellbarer Polymerer, z.B. Pulvern mit einer Korngröße kleiner 100 µm, in eine verwertbare Form ist in der DE-A-40 21 847 beschrieben, dieses Verfahren, das die Durchführung einer Polymerisationsreaktion beinhaltet, ist allerdings aufwendig.

Aufgabe der vorliegenden Erfindung ist es, praktisch als Absorptionsmaterial eingesetzte pulverförmige wasserquellbare hydrophile Polymere in einer einfach erhältlichen Form bereitzustellen, die die durch den Feinstaub verursachten Nachteile nicht aufweist, aber unverändert gute Performance und Verarbeitungseigenschaften aufweist.

Überraschend wurde nun gefunden, daß solche Absorptionsmaterialien erhältlich sind, indem pulverförmigen wasserquellbaren Polymeren geringe Mengen bestimmter Hilfsstoffe zugesetzt werden, durch die die Feinst- und Feinstaubpartikel im Sinne einer Agglomeration und/oder Verklebung dauerhaft an die gröberen Partikel des Polymers gebunden werden.

Gegenstand der vorliegenden Erfindung ist ein pulverförmiges wasserquellbares hydrophiles (Co-)Polymer, dadurch gekennzeichnet, daß das Pulver zur Verringerung des Staubens einen oder mehrere Hilfsstoffe aus der Reihe der Silicone enthält und/oder einen oder mehrere andere Hilfsstoffe, letztere in einer Gesamtmenge von weniger als 1 Gew.%, bezogen auf das Gewicht des (Co-)Polymer-Pulvers, aus der Reihe der Fettalkohole, Fettalkoholester, Fettsäuren, Fettsäureester, Fettsäureamide, sulfatierten Fettsäureamide und -ester, Sulfobernsteinsäureester, Polyole, Polyether, Polyglykole, Polyglykolether, aliphatischen Kohlenwasserstoffe und Paraffinöle enthält, wobei der Dampfdruck der einzelnen Hilfsstoffe bei 20°C höchstens 0,1 mbar beträgt und ihr Schmelzpunkt maximal bei 100°C liegt, mit der Maßgabe, daß, wenn der Hilfsstoff ein Polyol, Polyetheralkohol oder Polyethylenglykol ist, seine Menge mehr als 0,5% bezogen auf das Gewicht des (Co-)Polymers beträgt.

Als wasserquellbare hydrophile (Co-)Polymere kommen natürliche, teilsynthetische und vollsynthetische Substanzen in Betracht. Bevorzugt sind teilsynthetische und vollsynthetische Substanzen, insbesondere anionische (Co-)Polymere auf Basis (Meth-)Acrylsäure, die in teilneutralisierter Form als Alkalisalze, insbesondere Natrium- und/oder Kaliumsalze, vorliegen. Es kann sich um Homo- und Co-Polymerisate handeln, die allein aus Acrylsäure und/oder Methacrylsäure erhältlich sind, aus diesen Monomeren zusammen mit einem oder mehreren anderen Monomeren oder allein aus einem oder mehreren anderen Monomeren, aber beispielsweise auch um aufgepfropfte anionische (Co-)Polymere, z.B. auf Basis (Meth-)Acrylsäure, in teilneutralisierter Form als Alkalisalz vorliegend, z.B. um Pfropfpolymerisate auf Stärke oder Cellulose oder Polysacchariden oder Derivaten hiervon oder auf Polyalkylenoxiden, wie Polyethylenoxiden oder Polypropylenoxiden. Sind Polyalkylenoxide, also Polymerisate aus Ethylenoxid und/oder Propylenoxid, Pfropfgrundlage, so können diese beispielsweise auch verethert oder verestert sein, z.B. als Di-maleinsäurehalbester vorliegen. Bei den Polymerisaten kann es sich also beispielsweise um die in der EP-A-316 792 oder der EP-A-400 283 beschriebenen handeln.

Als Beispiele für Monomere, die neben (Meth-)Acrylsäure bei der Herstellung der Polymerisate Verwendung finden können, seien (Meth-)Acrylsäuremethyl-, -ethyl- und -hydroxyalkylester, (Meth-)Acrylamid, Crotonsäure, Malein-und Fumarsäure, Itaconsäure, 2-Acrylamido-2-methylpropansulfonsäure, Vinylsulfonsäure und Vinylphosphonsäure und die Methyl-, Ethyl-und Hydroxyalkylester und Amide dieser Säuren, amino- und ammoniumgruppenhaltige Ester und Amide aller genannten Säuren und wasserlösliche N-Vinylamide genannt, aber auch aus allen weiteren, bei der Herstellung von Superabsorber-Polymerisaten üblichen Monomeren stammende Baueinheiten können im Polymerisat enthalten sein. Das Polymerisat ist bevorzugt vernetzt. Als Beispiele für geeignete, bei der Herstellung der Superabsorber-Polymerisate.einsetzbare, zwei oder mehr olefinische Doppelbindungen enthaltende Vernetzersubstanzen, deren Bauelemente dann im (Co-)-Polymer enthalten sein können, seien Methylenbis(meth-)-acrylamid, Bisacrylamidoessigsäure, Ester ungesättigter Säuren von Polyolen, z.B. Ethylenglykoldi(meth-)acrylat oder Trimethylolpropantriacrylat oder Allylverbindungen, wie z.B. Allyl(meth-)acrylat, Polyallylester, Tetraallyloxyethan, Triallylamin, Tetraallylethylendiamin oder Allylester der Phosphorsäure sowie Vinylphosphonsäurederivate, wie sie beispielsweise in der EP-A-343 427 beschrieben sind, genannt. Der Anteil an Vernetzern, die bereits bei der Herstellung der Superabsorber-Polymerisate zugegeben sind, liegt bevorzugt bei 0 bis 20 Gew.%, besonders bevorzugt bei 0 bis 3 Gew.%, bezogen auf die eingesetzten Gesamt-Monomeren. Die Herstellung des (Co-)-Polymers erfolgt ansonsten nach an sich bekannten Methoden, wie sie z.B. in der EP-A-400 283 beschrieben sind, bevorzugt durch Polymerisation in wäßriger Lösung nach dem Verfahren der sogenannten Gelpolymerisation. Die Superabsorber-Polymerisate können auch in an sich bekannter Weise in wäßriger Gelphase nachvernetzt worden sein.

Auch die Zerkleinerung, Trocknung und Mahlung des Superabsorber-Polymerisats, also die Herstellung des erfindungsgemäß bezüglich seiner Staubbildung zu verbessernden (Co-)Polymer-Pulvers, erfolgt nach an sich bekannten Methoden. Die durch Mahlung erhaltenen Superabsorber-Polymerisat-Partikel können auch oberflächenvernetzt sein. Bei den erfindungsgemäßen staubarmen (Co-)Polymer-Pulvern handelt es sich bevorzugt um oberflächenvernetzte Polymere. Für eine solche Oberflächenvernetzung geeignete Substanzen sind z.B. Verbindungen, die zwei oder mehr Gruppen enthalten, die mit den Carboxylgruppen des hydrophilen Polymeren kovalente Bindungen bilden können. Geeignete Verbindungen sind beispielsweise Di- und Polyglycidylverbindungen, wie Phosphonsäurediglycidylester, Alkoxysilylverbindungen, Polyaziridine, Polyamine oder Polyamidoamine, wobei die genannten Verbindungen auch in Mischungen untereinander verwendet werden können (siehe beispielsweise EP-A-83 022, EP-A-543 303 und EP-A-530 438). Als Vernetzer geeignete Polyamidoamine sind insbesondere in der EP-A-349 935 beschrieben.

Bevorzugt werden aus solchen durch Mahlung erhaltenen Pulvern erfindungsgemäße Superabsorber-(Co-)Polymer-Pulver, die Hilfsstoffe zur Verringerung des Staubens enthalten, hergestellt, die eine für die vorgesehene Verwendung geeignete Korngrößenverteilung haben, also z.B. zu 50-100 % die Kornfraktion 100 bis 850 µm, bevorzugt zu 70-100 %, besonders bevorzugt zu 90-100 % diese Kornfraktion enthalten.

Die Hilfsstoffe, die in den erfindungsgemäßen (Co-)Polymer-Pulvern zur Verringerung des Staubens enthalten sind, können bei Raumtemperatur flüssig oder fest sein. Bevorzugt sind Hilfsstoffe, die bei Raumtemperatur als Flüssigkeit vorliegen.

In den erfindungsgemäßen (Co-)Polymer-Pulvern sind bevorzugt Silicone enthalten. Geeignete Hilfsstoffe aus der Reihe der Silicone sind z.B. Polysiloxane, in denen als organische Reste Methyl-, Ethyl-, Propyl- oder Phenylgruppen, auch gleichzeitig mehrere dieser Reste, enthalten sind. Bevorzugt sind Polydimethylsiloxane und Polymethylphenylsiloxane, besonders bevorzugt Polydimethylsiloxane. Die Polysiloxane können Ketten- und ringförmige Polymere sein, bevorzugt sind solche mit linearem Aufbau, insbesondere Polydimethylsiloxane mit linearem Aufbau. Bevorzugt ist es weiterhin, die Silicone oder Polysiloxane in Form der handelsüblichen Produkte einzusetzen, die üblicherweise ein Substanzgemisch darstellen und auch modifizierte Silicone sein können. Als handelsübliche flüssige Silicone sind die allgemein als Siliconöle bezeichneten Produkte bevorzugt, insbesondere wiederum Siliconöle auf Basis Polydimethylsiloxan, speziell mit linearem Aufbau. Bevorzugt sind schließlich Siloxane mit einer Viskosität bei 25°C von 5 bis 20000 cSt, besonders bevorzugt solche mit 0,5 bis 3,5 cm³/s (50 bis 350 cSt), ganz besonders bevorzugt solche mit 0,8 bis 1,2 cm³/s (80 bis 120 cSt), speziell solche mit ca. 1 cm³/s (100 cSt).

Beispiele für andere Hilfsstoffe, die allein oder als Gemisch mehrerer dieser Stoffe, gewünschtenfalls auch neben einem oder mehreren Siliconen, in einer Gesamtmenge von weniger als 1 Gew.%, bezogen auf das Gewicht des (Co-)Polymer-Pulvers, zur Verringerung des Staubens in den erfindungsgemäßen (Co-)Polymeren enthalten sein können, sind Hexadecanol, Octadecanol, Hexadecylacetat, Octadecylacetat, Palmitinsäure, Stearinsäure, Palmitinsäuremethylester, Stearinsäuremethylester, Sulfobernsteinsäurediisooctylester in Form seines Natriumsalzes, Hexylenglykol, 2-Methyl-2,4-pentandiol, Octamethylenglykol, Octadecan, Eicosan, handelsübliche Paraffinöle und Paraffine, die z.B. paraffinische, naphthenische und aromatische Kohlenwasserstoffe enthalten können, mit einem Schmelzpunkt von maximal 100°C und einem Dampfdruck von höchstens 0,1 mbar bei 20°C.

Bevorzugt sind als nicht zu den Siliconen gehörende andere Hilfsstoffe zur Verringerung des Staubens Polyglykole und ihre Derivate, insbesondere Polyalkylenglykole und Polyalkylenglykolether, vor allem die Mono- und Dialkylether. Besonders bevorzugt sind Polyethylenglykole, Polypropylenglykole, Ethylenoxid/Propylenoxid-Mischpolymerisate, insbesondere Blockpolymerisate, Polyethylenglykol- und Polypropylenglykol-mono- und -di-(C₁-C₄)alkylether, insbesondere Methylether, aber auch Polyglykolether von höhermolekularen Fettalkoholen, mit einem Molekulargewicht, aufgrund dessen die Anforderungen an den Dampfdruck und Schmelzpunkt erfüllt sind. Bevorzugt ist es wiederum, die Polyglykole und Polyglykolether in Form handelsüblicher Produkte einzusetzen, die üblicherweise ein Gemisch verschiedener Substanzen, insbesondere solcher mit verschiedenen Molekulargewichten, darstellen.

Die gewünschte Verringerung des Staubens der Superabsorber-Pulver wird bereits mit niedrigsten Einsatzmengen der angegebenen Hilfsstoffe erzielt. Sind als Hilfsstoffe Silicone im (Co-)Polymer-Pulver enthalten, so können diese auch in einer Menge von z.B. bis zu 5 Gew.%, bezogen auf das Gewicht des (Co-)Polymers, enthalten sein, ohne daß wesentliche nachteilige Änderungen anderer Eigenschaften des Pulvers auftreten. Bevorzugt sind Silicone in einer Gesamtmenge von 0,005 bis 5,0 Gew.%, besonders bevorzugt 0,01 bis 5,0 Gew.%, ganz besonders bevorzugt 0,01 bis 1,0 Gew.%, jeweils bezogen auf das Gewicht des (Co-)Polymer-Pulvers, enthalten. Sind ein oder mehrere andere Hilfsstoffe - allein oder zusammen mit Siliconen - zur Staubverminderung enthalten, so beträgt die Gesamtmenge dieser anderen Hilfsstoffe, bezogen auf das Gewicht des (Co-)Polymer-Pulvers, bevorzugt 0,01 bis 0,95 Gew.%, besonders bevorzugt 0,05 bis 0,7 Gew.%, ganz besonders bevorzugt 0,05 bis 0,5 Gew.%.

Die Herstellung des (Co-)Polymer-Pulvers mit verringertem Stauben kann in an sich bekannte Weise z.B. dadurch erfolgen, daß das Superabsorber-Pulver innig und möglichst homogen mit dem oder den Hilfsstoffen, gewünschtenfalls auch gleichzeitig mit weiteren Hilfsstoffen für andere Zwecke, z.B. Substanzen, die für die oben erwähnte Oberflächenvernetzung geeignet sind, vermischt wird. Das Mischen kann in einer kontinuierlichen oder diskontinuierlichen Arbeitsweise erfolgen in jeder zur Mischung von pulverförmigen Produkten mit festen oder flüssigen Zusätzen geeigneten Apparatur, beispielsweise in einem Taumelmischer. Bevorzugt ist es, das pulverförmige wasserquellbare hydrophile (Co-)Polymer mit verringertem Stauben herzustellen, indem das (Co-)Polymer mit dem oder den Hilfsstoffen in flüssiger Form oder mit einer flüssigen Lösung oder Dispersion des oder der Hilfsstoffe beaufschlagt wird. Der oder die flüssigen Hilfsstoffe bzw. die flüssige Lösung oder Dispersion des oder der Hilfsstoffe wird dabei gleichmäßig dem Superabsorber-Pulver zugesetzt, bevorzugt aufgesprüht, und mit diesem innig vermischt. Dies kann beispielsweise in einem Peterson-Kelly-Mischer erfolgen oder z.B. nach dem in der EP-A-534 226 beschriebenen Verfahren. Wird der oder die Hilfsstoffe in Form einer Lösung zugesetzt, so ist es bevorzugt, das Lösungsmittel nach dem Vermischen wieder abzudestillieren, d.h. nochmals eine Trocknung durchzuführen. Geeignete Lösungsmittel für die Beaufschlagung mit den Hilfsstoffen zur Verringerung des Staubens sind beispielsweise Alkohole, wie z.B. Methanol, Isopropanol oder Propandiol-1,2, Ketone, wie z.B. Methylethylketon, oder Ester, wie Essigsäureethylester oder Essigsäure-n-, -iso-, -sek.- und -tert.-Butylester. Die Beaufschlagung des (Co-)Polymers mit dem oder den Hilfsstoffen findet bevorzugt im Temperaturbereich von 0°C bis 100°C, besonders bevorzugt im Bereich von 10°C bis 80°C, ganz besonders bevorzugt im Bereich von 20°C bis 40°C statt.

Gegenstand der vorliegenden Erfindung ist aber auch generell ein Verfahren zur Verringerung des Staubens pulverförmiger wasserquellbarer hydrophiler (Co-)Polymerer, dadurch gekennzeichnet, daß das Pulver mit einem oder mehreren Hilfsstoffen aus der Reihe der Silicone beaufschlagt wird und/oder mit einem oder mehreren anderen Hilfsstoffen, letztere in einer Gesamtmenge von weniger als 1 Gew.%, bezogen auf das Gewicht des (Co-)Polymer-Pulvers, aus der Reihe der Fettalkohole, Fettalkoholester, Fettsäuren, Fettsäureester, Fettsäureamide, sulfatierten Fettsäureamide und -ester, Sulfobernsteinsäureester, Polyole, Polyether, Polyglykole, Polyglykolether, aliphatischen Kohlenwasserstoffe und Paraffinöle beaufschlagt wird, wobei der Dampfdruck der einzelnen Hilfsstoffe bei 20°C höchstens 0,1 mbar beträgt und ihr Schmelzpunkt maximal bei 100°C liegt. Bevorzugt sind Ausführungsformen dieses Verfahrens, die den oben als bevorzugt bezeichneten Varianten der Erfindung entsprechen.

Die vorliegende Erfindung betrifft weiterhin die Verwendung von Substanzen aus der Reihe der Silicone, allein oder in Mischungen, als Hilfsstoffe zur Verringerung des Staubens von pulverförmigen wasserquellbaren hydrophilen (Co-)Polymeren und die Verwendung, allein oder in Mischungen und in einer Gesamtmenge von weniger als 1 Gew.%, bezogen auf das Gewicht des (Co-)Polymer-Pulvers, von Substanzen aus der Reihe der Fettalkohole, Fettalkoholester, Fettsäuren, Fettsäureester, Fettsäureamide, sulfatierten Fettsäureamide und -ester, Sulfobernsteinsäureester, Polyole, Polyether, Polyglykole, Polyglykolether, aliphatischen Kohlenwasserstoffe und Paraffinöle als Hilfsstoffe zur Verringerung des Staubens von pulverförmigem wasserquellbaren hydrophilen (Co-)Polymeren, wobei der Dampfdruck der derart verwendeten einzelnen Substanzen bei 20°C höchstens 0,1 mbar beträgt und ihr Schmelzpunkt maximal bei 100°C liegt. Bevorzugte Varianten dieser Verwendung sind auch hier diejenigen, die den oben als bevorzugt bezeichneten Varianten der Erfindung entsprechen.

Bei der erfindungsgemäßen Art der Staubbindung bei pulverförmigen wasserquellbaren hydrophilen (Co-)Polymeren wird dieser Effekt bereits mit geringen Einsatzmengen der Hilfsstoffe in hervorragender Weise erzielt. Vielfach sind bereits Einsatzmengen von 0,05 oder 0,1 Gew.%, meistens Mengen unter 0,5 Gew.%, bezogen auf das Gewicht des (Co-)Polymer-Pulvers ausreichend, um die gewünschte Verringerung des Feinststaub- bzw. Feinstaubanteils im Superabsorber zu erzielen und z.B. die Handlingsprobleme im Produktions- und Verarbeitungsbetrieb zu lösen, so daß dort in kostengünstiger Weise der Absaugaufwand verringert werden kann. Durch diese geringen Mengen an Hilfsstoffen zur Verringerung des Staubens wird im allgemeinen keine Verschlechterung anderer Eigenschaften der Superabsorber-Pulver bewirkt, etwa eine Abnahme der Rieselfähigkeit oder eine Abnahme des Schüttgewichts. Prinzipiell wird jedoch in einem z.B. vom Hilfsstoff, vom (Co-)Polymer und vom Mischungsverfahren abhängigen unterschiedlichen Ausmaß bei größeren Einsatzmengen der Hilfsstoffe eine für viele Verwendungszwecke unerwünschte Abnahme z.B. des Schüttgewichts und der Rieselfähigkeit der Superabsorber-Pulver beobachtet. Bekannte Superabsorber-Abmischungen, die entsprechende Hilfsstoffe zur Erzielung der dort angestrebten Effekte in größeren Mengen enthalten, weisen vielfach solche Nachteile auf.

Besonders überraschend ist es, daß in der Regel nicht nur eine effiziente Staubbindung bereits mit einer solch geringen Einsatzmenge der Hilfsstoffe erzielt wird, durch die z.B. die Schüttguteigenschaften des Produkts nicht nachteilig verändert werden, sondern daß Superabsorber-Pulver, denen als Hilfsstoffe zur Verringerung des Staubens Silicone zugesetzt wurden, sogar ein höheres Schüttgewicht aufweisen, was auf eine Entlüftung des Raums zwischen den Polymerpartikeln und eine dadurch erzielte höhere Packungsdichte im Polymerpulver zurückzuführen sein dürfte. Insbesondere bei den als Hilfsstoffen bevorzugten Polydimethylsiloxanen, insbesondere denen mit linearem Aufbau und ganz speziell bei denen mit einer Viskosität von 0,5 bis 3,5 cm³/s (50 bis 350 cSt) bei 25°C, z.B. ca. 1 cm³/s (100 cSt) bei 25°C, wird über die schon mit niedrigsten Einsatzmengen erzielte Staubbindung hinaus selbst z.B. bei Einsatzmengen von 5 % nicht nur keine signifikante Abnahme der Schüttguteigenschaften, sondern im Gegenteil eine Anhebung des Schüttgewichts registriert.

Die erfindungsgemäßen, Hilfsstoffe zur Verringerung des Staubens enthaltenden pulverförmigen wasserquellbaren hydrophilen (Co-)Polymeren können für alle Zwecke verwendet werden, für die solche Superabsorber üblicherweise eingesetzt werden, insbesondere also zur Absorption von Wasser und wäßrigen Lösungen. Bevorzugt finden sie verwendung bei der Absorption von Körperflüssigkeiten, insbesondere von Blut und Urin. Hierzu werden sie insbesondere in absorbierende Einweg-Hygieneartikel, z.B. in Windeln, Tampons oder Damenbinden oder für andere medizinische Zwecke, eingearbeitet. Andere Verwendungszwecke sind z.B. die als wasserspeichernde Bodenverbesserungsmittel oder als Feuchtigkeitsbindemittel bei der Kabelummantelung.

### Beispiele

Die in den Beispielen angegebenen Abkürzungen haben die folgenden Bedeutungen:
- PEG:: Polyethylenglykol mit einem durchschnittlichen Molekulargewicht entsprechend der angegeben Zahl
- MPG:: Methylpolyethylenglykol mit einem durchschnittlichen Molekulargewicht entsprechend der angegebenen Zahl
- MTG:: Methyltriethylenglykol
- HEXG:: Hexylenglykol (2-Methyl-2,4-pentandiol)
- PAFF:: Paraffinöl technisch ^{®}PIONIER 2024, kommerzielles Produkt der Fa. Hansen & Rosenthal, Hamburg (^{®}PIONIER ist ein eingetragenes Warenzeichen der Firma Hansen & Rosenthal)
- SILI:: Polydimethylsiloxan, kommerzielles Produkt der Bayer AG mit dem Handelsnamen ^{®}BAYSILONE M 100 (^{®}BAYSILONE ist ein eingetragenes Warenzeichen der Bayer AG)
- GENA:: Ethylenoxid/Propylenoxid-Blockpolymer, kommerzielles Produkt der Hoechst AG mit dem Handelsnamen ^{®}GENAPOL PF 40 (^{®}GENAPOL ist ein eingetragenes Warenzeichen der Hoechst AG)
- SUCC:: Natriumsalz des Sulfobernsteinsäurediisooctylesters, 70gew.%ig, gelöst in 1,2-Propandiol

### Beispiel 1:

500 g des unter dem Handelsnamen ^{®}SANWET IM 5000 S (^{®}SANWET ist ein eingetragenes Warenzeichen der Sanyo Chemical Industries) von der Hoechst AG/ Cassella AG kommerziell erhältlichen pulverförmigen wasserquellbaren Polymeren wurden mit 0,25 g Polyethylenglykol mit einem durchschnittlichen Molekulargewicht von 300 versetzt und in einer Flasche auf einer Rollvorrichtung durch vierstündiges kontinuierliches gleichmäßiges Rollen homogen vermischt. Der Feinstaubanteil im erhaltenen Superabsorber-Pulver und im unbehandelten Ausgangs-Polymerpulver ist in Tabelle 1 angegeben.

Die Bestimmung der Staubanteile erfolgte über eine Laser-Partikelgrößenanalyse nach dem Trockendispergierverfahren nach vorheriger Heraussiebung der Partikel mit einer Korngröße von größer 250 µm, d.h. Absiebung über ein Sieb mit einer Maschenweite von 250 µm. Diese Bestimmungsmethode eignet sich allerdings nur für Relativmessungen zu Vergleichszwecken, da sie, bedingt durch die Meßmethodik, zu hohe Werte für die untere Partikelgrößen angibt. Durch den Dispergiervorgang wird die Probe einer hohen Scherbelastung unterworfen, wodurch Abrieb und Zerkleinerung größerer Partikel auftritt.

### Beispiele 2 bis 15

Analog zu Beispiel 1 wurden mit weiteren Hilfsstoffen und anderen Hilfsstoffmengen erfindungsgemäße Superabsorber-Pulver mit verringertem Stauben hergestellt. Polymere, Hilfsstoffe, Einsatzmengen und Feinstaubanteile sind in Tabelle 1 angegeben.

**Tabelle 1**

| Beispiel | Polymer | Hilfsstoff | | Feinstaubanteil²⁾(%) |
|---|---|---|---|---|
| | | Typ | Menge¹⁾(%) | |
| - | ^{®}SANWET IM 5000 S | - | - | 0,095³⁾ |
| 1 | " | PEG 300 | 0,05 | 0,078³⁾ |
| 2 | " | " | 0,1 | 0,074³⁾ |
| 3 | " | " | 0,3 | 0,062³⁾ |
| 4 | " | " | 0,5 | 0,004³⁾ |
| 5 | " | MPG 350 | 0,1 | 0,073³⁾ |
| 6 | " | " | 0,3 | 0,068³⁾ |
| 7 | " | " | 1,0 | 0,019³⁾ |
| - | SANWET VS 3790 | - | - | 0,34⁴⁾ |
| 8 | " | HEXG | 0,05 | 0,28⁴⁾ |
| 9 | " | " | 0,07 | 0,15⁴⁾ |
| 10 | " | " | 0,1 | 0,18⁴⁾ |
| 11 | " | MTG | 0,05 | 0,28⁴⁾ |
| 12 | " | " | 0,07 | 0,24⁴⁾ |
| 13 | " | " | 0,1 | 0,21⁴⁾ |
| 14 | " | PAFF | 0,01 | 0,31⁴⁾ |
| 15 | " | " | 0,5 | 0,19⁴⁾ |

| | | | | |
|---|---|---|---|---|
| 1) Einsatzmenge in Gewichtsprozent, bezogen auf das Gewicht des eingesetzten Polymers | | | | |
| 2) Anteil des Feinstaubs mit einer Korngröße kleiner 10 µm im Superabsorber-Pulver, angegeben in Gewichtsprozent | | | | |
| 3) Bestimmt nach dem Trockendispergierverfahren | | | | |
| 4) Bestimmt nach dem Trockendispergierverfahren wie bei ³⁾, allerdings wurde in diesen Fällen ohne Absiebung der Kornfraktion größer 250 µm direkt die telquel-Ware vermessen. Die Ergebnisse sind reproduzierbar und für Vergleichs zwecke geeignet, aber absolut gesehen zu ungenau, da eine Meßbereichsüberschreitung durch zu große Partikel vorliegt. | | | | |

### Beispiele 16 bis 25

Die Herstellung des Polymers erfolgte nach folgender Vorschrift:

In einem durch geschäumtes Kunststoffmaterial gut isolierten Polyethylengefäß mit einem Fassungsvermögen von 10 l wurden 4950 g E-Wasser/Eis vorgelegt, 553 g Natriumbicarbonat darin suspendiert und langsam 1986 g Acrylsäure so zudosiert, daß ein Überschäumen der Reaktionslösung vermieden wurde, wobei sich diese auf eine Temperatur von ca. 5-3°C abkühlte. Es wurden 1,5 g eines Polyethylenglykol-300-di-Maleinsäurehalbesters zugegeben sowie 4,7 g Tetraallyloxyethan, 2,7 g Methylenbisacrylamid, 3,9 g Sorbitanmonolaurat und 22 g Harnstoff. Bei einer Temperatur von 4°C wurden die Initiatoren, ein Redoxsystem, bestehend aus 1,7 g 2,2'-Azobis-amidinopropan-dihydrochlorid, gelöst in 20 g E-Wasser, 3,9 g Kaliumperoxodisulfat, gelöst in 150 g E-Wasser, sowie 0,33 g Ascorbinsäure, gelöst in 20 g E-Wasser, nacheinander zugegeben und gut verrührt. Die Reaktionslösung wurde daraufhin ohne Rühren stehen gelassen, wobei durch die einsetzende Polymerisation, in deren Verlauf die Temperatur bis auf ca. 85°C anstieg, ein festes Gel entstand, das anschließend mechanisch zerkleinert wurde, mit 970 g Natronlauge 50%ig (Neutralisationsgrad der Acrylsäure = 73 Mol-%) versetzt und zweimal durchgeknetet wurde, mit 212 g eines auf 5% verdünnten handelsüblichen Polyamidoamins auf Basis Adipinsäure, Diethylentriamin, Ethylendiamin und Ethanolamin, das mit Epichlorhydrin umgesetzt und einer Alkali-Amin-Behandlung unterzogen worden war, versetzt und wiederum zweimal durchgeknetet wurde und anschließend bei Temperaturen über 150°C im Luftstrom getrocknet, gemahlen und gesiebt wurde.

In einem 1-1-Labor-Taumelmischer wurden jeweils 400 g dieses Polymerpulver vorgelegt. Nach Versetzen mit den in Tabelle 2 angegebenen Hilfsstoffen in den dort angegebenen Mengen ließ man zwei Stunden taumeln und bestimmte die Feinstaubanteile nach dem Trockendispergierverfahren.

**Tabelle 2**

| Beispiel | | Hilfsstoff | | Feinstaubanteil²⁾(%) |
|---|---|---|---|---|
| | | Typ | Menge¹⁾(%) | |
| - | unbehandelt | - | - | 0,885⁴⁾ |
| 16 | | SILI | 0,005 | 0,852⁴⁾ |
| 17 | | " | 0,03 | 0,700⁴⁾ |
| 18 | | " | 0,5 | 0,625⁴⁾ |
| 19 | | " | 1,0 | 0,475⁴⁾ |
| 20 | | " | 2,0 | 0,335⁴⁾ |
| 21 | | " | 3,0 | 0,276⁴⁾ |
| 22 | | " | 4,0 | 0,266⁴⁾ |
| 23 | | | 5,0 | 0,260⁴⁾ |
| 24 | | GENA | 0,5 | 0,730⁴⁾ |
| 25 | | SUCC | 0,5 | 0,440⁴⁾ |

| | | | | |
|---|---|---|---|---|
| 1), 2), 4) = siehe Tabelle 1 | | | | |

### Beispiele 26 bis 30

In einem großtechnischen Vibrationsmischer wurden die Hilfsstoffe während des Mischvorgangs dem Superabsorber-Polymer über die Schnecken zudosiert und der Mischvorgang bis zur Homogenität fortgeführt (Polymer, Hilfsstoff und Mengen siehe Tabelle 3). Die Bestimmung des Staubanteils erfolgte nach der "Fallmethode" in einem D.P.A.-Staubabscheider der Fa. Roaches (zur Staubanteilsbestimmung empfohlen nach Dokument ISO/TC 38/SC 1-N 646). Diese Methode ergibt genauere Werte bezüglich der Freisetzbarkeit der Feinstaubpartikel. Dabei fällt eine bestimmte Menge des pulverförmigen Produkts aus einer bestimmten Höhe in einen Behälter. Nach einer definierten Zeit wird der entstehende Staub unter kontrollierten Vakuumbedingungen abgesaugt und einem Laser-Analyzer zugefügt. Die Meßergebnisse sind in Tabelle 3 angegeben.

**Tabelle 3**

| Beispiel | Polymer | Hilfsstoff | | Feinstaubanteil⁵⁾ (ppm) |
|---|---|---|---|---|
| | | Typ | Menge¹⁾(%) | |
| - | SANWET IM 5000 SG | - | - | 121 |
| 26 | " | PEG 300 | 0,1 | 2 |
| - | SANWET IM 3900 G | - | - | 74 |
| 27 | " | PEG 300 | 0,05 | 5 |
| 28 | " | SILI | 0,05 | 2 |
| 29 | " | SILI + PEG300 | 0,005 + 0,03 | 3 |
| 30 | " | SILI + PAFF | 0,03 + 0,05 | 3 |

| | | | | |
|---|---|---|---|---|
| 1) = siehe Tabelle 1 | | | | |
| 5) Anteil des Feinstaubs mit einer Korngröße kleiner 10 µm im Superabsorber-Pulver, angegeben in mg Feinstaub/kg telquel-Produkt (= ppm) | | | | |

## Patentansprüche

1. Pulverförmiges wasserquellbares hydrophiles (Co-)Polymer, dadurch gekennzeichnet, daß das Pulver zur Verringerung des Staubens einen oder mehrere Hilfsstoffe aus der Reihe der Silicone enthält und/oder einen oder mehrere andere Hilfsstoffe, letztere in einer Gesamtmenge von weniger als 1 Gew.%, bezogen auf das Gewicht des (Co-)-Polymer-Pulvers, aus der Reihe der Fettalkohole, Fettalkoholester, Fettsäuren, Fettsäureester, Fettsäureamide, sulfatierten Fettsäureamide und -ester, Sulfobernsteinsäureester, Polyole, Polyether, Polyglykole, Polyglykolether, aliphatischen Kohlenwasserstoffe und Paraffinöle enthält, wobei der Dampfdruck der einzelnen Hilfsstoffe bei 20 °C höchstens 0,1 mbar beträgt und ihr Schmelzpunkt maximal bei 100 °C liegt, mit der Maßgabe, daß, wenn der Hilfsstoff ein Polyol, Polyetheralkohol oder Polyethylenglykol ist, seine Menge mehr als 0,5 % bezogen auf das Gewicht des (Co-)Polymerpulvers beträgt.

2. (Co-)Polymer-Pulver gemäß Anspruch 1, dadurch gekennzeichnet, daß es sich um ein anionisches (Co-)Polymer auf Basis (Meth-)Acrylsäure, in teilneutralisierter Form als Alkalisalz vorliegend, handelt.

3. (Co-)Polymer-Pulver gemäß Anspruch 1 und/oder 2, dadurch gekennzeichnet, daß es sich um ein aufgepfropftes anionisches (Co-)Polymer auf Basis (Meth-)Acrylsäure, in teilneutralisierter Form als Alkalisalz vorliegend, handelt.

4. (Co-)Polymer-Pulver gemäß einem oder mehreren der Ansprüche 1 bis 3, dadurch gekennzeichnet, daß ein oder mehrere Hilfsstoffe aus der Reihe der Polysiloxane enthalten sind.

5. (Co-)Polymer-Pulver gemäß Anspruch 4, dadurch gekennzeichnet, daß der Hilfsstoff ein Siliconöl mit einer Viskosität bei 25 °C von 0,5 bis 3,5 cm³/s ist.

6. (Co-)Polymer-Pulver gemäß einem oder mehreren der Ansprüche 1 bis 3, dadurch gekennzeichnet, daß ein oder mehrere Hilfsstoffe aus der Reihe der Polyalkylenglykole und der Polyalkylenglykolether enthalten sind.

7. (Co-)Polymer-Pulver gemäß einem oder mehreren der Ansprüche 1 bis 6, dadurch gekennzeichnet, daß es zur Verringerung des Staubens einen oder mehrere Hilfsstoffe aus der Reihe der Silicone in einer Gesamtmenge von 0,005 bis 5,0 Gew. % bezogen auf das Gewicht des (Co-)Polymer-Pulvers, und/oder einen oder mehrere andere Hilfsstoffe, letzere in einer Gesamtmenge von 0,01 bis 0,95 Gew. % bezogen auf das Gewicht des (Co-)Polymer-Pulvers, enthält.

8. Verfahren zur Herstellung eines pulverförmigen wasserquellbaren hydrophilen (Co-)Polymers mit verringertem Stauben gemäß einem oder mehreren der Ansprüche 1 bis 7, dadurch gekennzeichnet, daß das (Co-)Polymer mit dem oder den Hilfsstoffen in flüssiger Form oder mit einer flüssigen Lösung oder Dispersion des oder der Hilfsstoffe beaufschlagt wird.

9. Verfahren gemäß Anspruch 8, dadurch gekennzeichnet, daß die Beaufschlagung des (Co-)Polymers mit dem oder den Hilfsstoffen im Temperaturbereich von 0 °C bis 100 °C stattfindet.

10. Verwendung pulverförmiger wasserquellbarer hydrophiler (Co-)Polymere gemäß einem oder mehreren der Ansprüche 1 bis 7 zur Absorption von Wasser oder wäßrigen Lösungen.

11. Verwendung gemäß Anspruch 10 zur Absorption von Körperflüssigkeiten, insbesondere Blut und Urin.

## Claims

1. Pulverulent water-swellable hydrophilic (co)polymer, characterized in that, to reduce dusting, the powder comprises one or more auxiliaries from the series consisting of silicones and/or one or more other auxiliaries, the latter in a total amount of less than 1 % by weight, based on the weight of the (co)polymer powder, from the series consisting of fatty alcohols, fatty alcohol esters, fatty acids, fatty acid esters, fatty acid amides, sulphated fatty acid amides and esters, sulphosuccinic esters, polyols, polyethers, polyglycols, polyglycol ethers, aliphatic hydrocarbons and paraffin oils, the vapour pressure of the individual auxiliaries being not more than 0.1 mbar at 20°C and their melting point being not more than 100°C, with the proviso that, if the auxiliary is a polyol, polyether alcohol or polyethylene glycol, its amount is more than 0.5 % based on the weight of the (co)polymer powder.

2. (Co)polymer powder according to Claim 1, characterized in that it is an anionic (co)polymer based on (meth)acrylic acid present in partly neutralized form as an alkali metal salt.

3. (Co)polymer powder according to Claim 1 and/or 2, characterized in that it is a grafted anionic (co)polymer based on (meth)acrylic acid present in partly neutralized form as an alkali metal salt.

4. (Co)polymer powder according to one or more of Claims 1 to 3, characterized in that it comprises one or more auxiliaries from the series consisting of polysiloxanes.

5. (Co)polymer powder according to Claim 4, characterized in that the auxiliary is a silicone oil having a viscosity at 25°C of 0.5 to 3.5 cm³/ₛ.

6. (Co)polymer powder according to one or more of Claims 1 to 3, characterized in that it comprises one or more auxiliaries from the series consisting of polyalkylene glycols and polyalkylene glycol ethers.

7. (Co)polymer powder according to one or more of Claims 1 to 6, characterized in that it comprises, for reducing dusting, one or more auxiliaries from the series consisting of silicones in a total amount of 0.005 to 5.0 % by weight based on the weight of the (co)polymer powder, and/or one or more other auxiliaries, the latter in a total amount of 0.01 to 0.95 % by weight based on the weight of the (co)polymer powder.

8. Process for the preparation of a pulverulent water-swellable hydrophilic (co)polymer with reduced dusting according to one or more of Claims 1 to 7, characterized in that the (co)polymer is charged with the auxiliary or auxiliaries in liquid form or with a liquid solution or dispersion of the auxiliary or auxiliaries.

9. Process according to Claim 8, characterized in that the charging of the (co)polymer with the auxiliary or auxiliaries takes place in the temperature range from 0°C to 100°C.

10. Use of pulverulent water-swellable hydrophilic (co)polymers according to one or more of Claims 1 to 7 for absorption of water or aqueous solutions.

11. Use according to Claim 10 for the absorption of body fluids, in particular blood and urine.

## Revendications

1. (Co)polymère hydrophile sous forme de poudre, pouvant gonfler dans l'eau, caractérisé en ce que la poudre contient un ou plusieurs adjuvants de la série des silicones pour réduire la poussière, et/ou un ou plusieurs autres adjuvants, ces derniers étant présents en une quantité totale inférieure à 1% en poids, par rapport au poids du (co)polymère en poudre, et étant de la série des alcools gras, des esters d'alcools gras, des acides gras, des esters d'acide gras, des amides d'acides gras, des amides et des esters d'acides gras sulfatés, des esters d'acide sulfosuccinique, des polyols, des polyéthers, des polyglycols, des polyglycoléthers, des hydrocarbures aliphatiques et des huiles de paraffine, où la pression de vapeur à 20°C des adjuvants seuls s'élève au plus à 0,1 mbar et leur point de fusion est de 100°C au maximum, étant spécifié que lorsque l'adjuvant est un polyol, un polyétheralcool ou un polyéthylèneglycol, sa teneur est supérieure à 0,5% en poids, par rapport au poids de la poudre de (co)polymère.

2. Poudre de (co)polymère selon la revendication 1, caractérisée en ce qu'il s'agit d'un (co)polymère anionique à base d'acide (méth)acrylique, se trouvant sous forme partiellement neutralisée en tant que sel alcalin.

3. Poudre de (co)polymère selon la revendication 1 et/ou 2, caractérisée en ce qu'il s'agit d'un (co)polymère anionique greffé à base d'acide (méth)acrylique, se trouvant sous forme partiellement neutralisée en tant que sel alcalin.

4. Poudre de (co)polymère selon l'une quelconque des revendications 1 à 3, caractérisée en ce qu'elle contient un ou plusieurs adjuvants de la série des polysiloxanes.

5. Poudre de (co)polymère selon la revendication 4, caractérisée en ce que l'adjuvant est une huile de silicone ayant une viscosité à 25°C de 0,5-3,5 cm³/s.

6. Poudre de (co)polymère selon l'une quelconque des revendications 1 à 3, caractérisée en ce qu'elle contient un ou plusieurs adjuvants de la série des polyalkylèneglycols et des polyalkylèneglycoléthers.

7. Poudre de (co)polymère selon l'une quelconque des revendications 1 à 6, caractérisée en ce qu'elle contient un ou plusieurs adjuvants de la série des silicones pour réduire la poussière en une quantité totale de 0,005-5,0% en poids, par rapport au poids de la poudre de (co)polymère, et/ou un ou plusieurs autres adjuvants, ces derniers étant présents en une quantité totale de 0,01-0,95% en poids, par rapport au poids de la poudre de (co)polymère.

8. Procédé de préparation d'un (co)polymère hydrophile sous forme de poudre, pouvant gonfler dans l'eau, produisant peu de poussière, selon l'une quelconque des revendications 1 à 7, caractérisé en ce que le (co)polymère est additionné du ou des adjuvants sous forme liquide ou avec une dispersion ou une solution liquide du ou des adjuvants.

9. Procédé selon la revendication 8, caractérisé en ce que l'ajout du ou des adjuvants au (co)polymère est effectué dans un domaine de température de 0-100°C.

10. Utilisation du (co)polymère hydrophile sous forme de poudre et pouvant gonfler dans l'eau, selon l'une quelconque des revendications 1 à 7 pour l'absorption d'eau ou de solutions aqueuses.

11. Utilisation selon la revendication 10 pour l'absorption liquides corporels, en particulier le sang et l'urine.
